# EUROPEAN PATENT APPLICATION

(11) **EP 3 815 704 A1**
(43) Date of publication of application: **05.05.2021**
(21) Application number: 19825186.0
(22) Date of filing: 28.06.2019
(51) Int. Cl.: A61K 38/05, A61K 9/08, A61K 9/14, A61K 47/02, A61K 47/14, A61K 47/26, A61K 47/34, A61P 27/02

(54) **FORMULATION CONTAINING EMRICASAN**

(30) Priority: 29.06.2018 JP 2018125234
(71) Applicant: The Doshisha, Kyoto-shi, Kyoto 602-8580 (JP)
(72) Inventor: KOIZUMI Noriko, Kyotanabe-shi, Kyoto 610-0394 (JP); OKUMURA Naoki, Kyotanabe-shi, Kyoto 610-0394 (JP)
(74) Representative: J A Kemp LLP
(86) International application number: PCT/JP2019/025945
(87) International publication number: WO 2020/004652

(57) **Abstract**

The present disclosure provides a formulation having improved solubility and/or stability of emricasan. In one embodiment, the present disclosure provides a formulation containing (i) emricasan or a derivative thereof, or a pharmaceutically acceptable salt thereof, or a solvate thereof, (ii) a nonionic surfactant including a polyoxyethylene group, and (iii) a buffering agent that maintains the pH at about 4.5 to about 8.5. The present disclosure also provides a composition in the form of powder for preparing such a formulation at the time of use.

## Description

### [Technical Field]

The present disclosure relates to a formulation containing emricasan or a derivative thereof as an active ingredient.

### [Background Art]

Emricasan is known as a pan caspase inhibitor and has an effect such as suppression of apoptosis. While emricasan dissolves well in an organic solvent such as ethanol or DMSO, emricasan is difficult to dissolve in water. Thus, it is difficult to use emricasan as a formulation which is dissolved in water such as an eye drop to be used. However, emricasan is expected to have effectiveness as a formulation for various diseases. Examples include Fuchs' endothelial corneal dystrophy (FECD), which is one of the diseases that disorder corneal endothelial cells. FECD is a disease wherein corneal endothelial cells are progressively reduced, and progression of FECD results in loss of vision. The only therapeutic method for FECD at present is corneal transplant. However, there is a need for development of a novel therapeutic method due to problems such as invasiveness of surgery and shortage in donors. Emricasan exhibits effectiveness for this disease in vitro (PTL1). In view of the foregoing, there is a great demand for a water-soluble formulation of emricasan.

### [Citation List]

### [Patent Literature]

[PTL 1] International Publication No. WO 2017/110094

### [Summary of Invention]

### [Solution to Problem]

The inventors have found conditions under which emricasan or a derivative thereof at a concentration which can be expected to achieve a pharmaceutical effect is solubilized in a solution (in particular, an aqueous solution). The inventors have also found conditions under which emricasan or a derivative thereof is stabilized in a solution (in particular, an aqueous solution). Specifically, the inventors have found that the soluble property and/or stability of emricasan or a derivative thereof in a solution is optimized by adjusting a solubilizing agent (e.g., a surfactant) and a pH condition in a formulation.

Accordingly, the present disclosure provides, for example, the following.
(Item 1) A formulation comprising:
   (i) emricasan or a derivative thereof, or a pharmaceutically acceptable salt thereof, or a solvate thereof;
   (ii) a nonionic surfactant comprising a polyoxyethylene group; and
   (iii) a buffering agent that maintains pH at about 3.0 to about 8.5.
(Item 2) The formulation of item 1, wherein the buffering agent maintains pH at about 3.5 to about 8.0.
(Item 3) The formulation of any one of the preceding items, wherein the buffering agent maintains pH at about 4.0 to about 7.0.
(Item 4) The formulation of any one of the preceding items, wherein the buffering agent maintains pH at about 4.5 to about 6.5.
(Item 5) The formulation of any one of the preceding items, wherein the surfactant is present in the formulation at about 0.05% by weight to about 5% by weight.
(Item 6) The formulation of any one of the preceding items, wherein the surfactant is present in the formulation at about 0.1% by weight to about 4% by weight.
(Item 7) The formulation of any one of the preceding items, wherein the nonionic surfactant comprising a polyoxyethylene group is selected from the group consisting of polyoxyethylene hydrogenated castor oil, polyoxyethylene castor oil, polyethylene glycol monostearate, polyoxyethylene sorbitan fatty acid ester, and polyoxyethylene-polyoxypropylene copolymer.
(Item 8) The formulation of any one of the preceding items, wherein the nonionic surfactant comprising a polyoxyethylene group is selected from the group consisting of HCO-20, HCO-40, HCO-60, HCO-80, HCO-100, CO-35, MYS-40, Tween 80, and poloxamer 407.
(Item 9) The formulation of any one of the preceding items, wherein the emricasan or a derivative thereof, or a pharmaceutically acceptable salt thereof, or a solvate thereof is present in the formulation at about 0.05% by weight to about 5% by weight.
(Item 10) The formulation of any one of the preceding items, wherein the emricasan or a derivative thereof, or a pharmaceutically acceptable salt thereof, or a solvate thereof is present in the formulation at about 0.1% by weight to about 1% by weight.
(Item 11) A formulation comprising emricasan, wherein the formulation comprises:
   (i) emricasan that is present in the formulation at about 0.1% by weight to about 1% by weight;
   (ii) a surfactant selected from the group consisting of HCO-20, HCO-40, HCO-60, HCO-80, HCO-100, CO-35, MYS-40, Tween 80, and poloxamer 407, the surfactant being present in the formulation at about 0.1% by weight to about 4% by weight; and
   (iii) a buffering agent that maintains pH at about 3.0 to about 8.0.
(Item 12) A formulation comprising emricasan, wherein the formulation comprises:
   (i) emricasan that is present in the formulation at about 0.1% by weight to about 1% by weight;
   (ii) HCO-60 that is present in the formulation at about 0.1% by weight to about 4% by weight; and
   (iii) a buffering agent that maintains pH at about 4.0 to about 7.0.
(Item 13) A formulation comprising emricasan, wherein the formulation comprises:
   (i) emricasan that is present in the formulation at about 0.1% by weight to about 1% by weight;
   (ii) Tween 80 that is present in the formulation at about 0.1% by weight to about 4% by weight; and
   (iii) a buffering agent that maintains pH at about 4.0 to about 7.0.
(Item 14) The formulation of the preceding item, wherein the formulation optionally further comprises a saccharide.
(Item 15) The formulation of the preceding items, wherein the saccharide is selected from the group consisting of mannitol, sucrose, and trehalose.
(Item 16) The formulation of any one of the preceding items, wherein the formulation is an ophthalmic formulation.
(Item 17) The formulation of item 16, wherein the formulation is a formulation for treating or preventing a corneal endothelial condition, disorder, or disease.
(Item 18) The formulation of any one of the preceding items, wherein the formulation is a formulation for instillation or a formulation for injection into an anterior chamber.
(Item 19) The formulation of any one of the preceding items, wherein the formulation is an aqueous solution.
(Item 20) A composition for preparing the formulation of any one of the preceding items at the time of use, comprising emricasan or a derivative thereof, or a pharmaceutically acceptable salt thereof, or a solvate thereof, wherein the composition is characterized by being in a powder form and being reconstituted with a medium for reconstitution.
(Item 21) The composition of any one of the preceding items, wherein the composition comprises:
   (i) emricasan or a derivative thereof, or a pharmaceutically acceptable salt thereof, or a solvate thereof;
   (ii) a nonionic surfactant comprising a polyoxyethylene group; and
   (iii) a buffering agent that maintains pH at about 3.0 to about 8.5.
(Item 22) The composition of any one of the preceding items, wherein the medium for reconstitution comprises purified water, saline, buffer, or any combination thereof.
(Item 23) The composition of any one of the preceding items, wherein the medium for reconstitution further comprises at least one selected from the group consisting of an antiseptic, a stabilizer, and an isotonicifier.
(Item 24) The composition of any one of the preceding items, wherein the powder form is a lyophilized powder form.
(Item 25) The composition of any one of the preceding items, wherein the medium for reconstitution comprises a nonionic surfactant comprising a polyoxyethylene group.
(Item 26) The composition of any one of the preceding items, wherein the medium for reconstitution comprises a buffering agent that maintains pH at about 3.0 to about 8.5.
(Item 27) The composition of the preceding items, wherein the medium for reconstitution optionally further comprises a saccharide.
(Item 28) The composition of any one of the preceding items, wherein the saccharide is selected from the group consisting of mannitol, sucrose, and trehalose.
(Item 29) A kit for preparing the formulation of any one of the preceding items at the time of use, wherein the kit comprises:
   (i) powder which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5; and
   (ii) a solvent for reconstitution.
(Item 30) The kit of any one of the preceding items, wherein the powder is powder obtained by lyophilizing a solution which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5.
(Item 31) The kit of any one of the preceding items, wherein the medium for reconstitution comprises purified water, saline, buffer, or any combination thereof.
(Item 32) The kit of any one of the preceding items, wherein the medium for reconstitution further comprises at least one selected from the group consisting of an antiseptic, a stabilizer, and an isotonicifier.
(Item 33) A kit for preparing the formulation of any one of the preceding items at the time of use, wherein the kit comprises:
   (i) powder of emricasan or a derivative thereof, or a pharmaceutically acceptable salt thereof, or a solvate thereof;
   (ii) optionally a nonionic surfactant comprising a polyoxyethylene group; and
   (iii) optionally a buffering agent that maintains pH at about 3.0 to about 8.5.
(Item 34) A kit for preparing the formulation of any one of the preceding items at the time of use, wherein the kit comprises:
   (i) powder of emricasan or a derivative thereof, or a pharmaceutically acceptable salt thereof, or a solvate thereof;
   (ii) a medium for reconstitution which comprises a nonionic surfactant comprising a polyoxyethylene group and a buffering agent that maintains pH at about 3.0 to about 8.5.
(Item 35) The kit of any one of the preceding items, wherein the powder is lyophilized powder.
(Item 36) The kit of any one of the preceding items, wherein the medium for reconstitution optionally further comprises a saccharide.
(Item 37) The kit of any one of the preceding items, wherein the saccharide is selected from the group consisting of mannitol, sucrose, and trehalose.
(Item 38) A method for preparing the formulation of any one of the preceding items at the time of use, comprising:
   (i) providing powder which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5; and
   (ii) reconstituting the powder with a medium for reconstitution.
(Item 39) The method of any one of the preceding items, wherein the powder is powder obtained by lyophilizing a solution which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5.
(Item 40) The method of any one of the preceding items, wherein the medium for reconstitution comprises purified water, saline, buffer, or any combination thereof.
(Item 41) The method of any one of the preceding items, wherein the medium for reconstitution further comprises at least one selected from the group consisting of an antiseptic, a stabilizer, and an isotonicifier.
(Item 42) A method for preparing the formulation of any one of the preceding items at the time of use, comprising:
   (i) providing powder of emricasan or a derivative thereof, or a pharmaceutically acceptable salt thereof, or a solvate thereof; and
   (ii) reconstituting the powder with a medium for reconstitution which comprises a nonionic surfactant comprising a polyoxyethylene group and a buffering agent that maintains pH at about 6.0 to about 8.5.
(Item 43) The method of any one of the preceding items, wherein the powder is lyophilized powder.
(Item 44) The method of any one of the preceding items, wherein the solvent for reconstitution optionally further comprises a saccharide.
(Item 45) The method of any one of the preceding items, wherein the saccharide is selected from the group consisting of mannitol, sucrose, and trehalose.
(Item 46) A medium or solution for reconstituting lyophilized powder of emricasan or a derivative thereof, or a pharmaceutically acceptable salt thereof, or a solvate thereof, or for dissolving a crystal or powder thereof of emricasan or a derivative thereof, or a pharmaceutically acceptable salt thereof, or a solvate thereof, wherein the medium or solution comprises:
   (i) a solvent;
   (ii) a nonionic surfactant comprising a polyoxyethylene group; and
   (iii) a buffering agent that maintains pH at about 3.0 to about 8.5.
(Item 47) The medium or solution of any one of the preceding items, wherein the medium or solution optionally further comprises a saccharide.
(Item 48) The medium or solution of any one of the preceding items, wherein the saccharide is selected from the group consisting of mannitol, sucrose, and trehalose.
(Item 49) A therapy of and/or method for treating a disease, disorder, or condition that can be treated and/or prevented by emricasan in a subject, comprising a process of administering an effective amount of a formulation comprising emricasan to the subject in need thereof, wherein the formulation comprises:
   (i) emricasan or a derivative thereof, or a pharmaceutically acceptable salt thereof, or a solvate thereof;
   (ii) a nonionic surfactant comprising a polyoxyethylene group; and
   (iii) a buffering agent that maintains pH at about 3.0 to about 8.5.
(Item 50) The method of item 49, further comprising a feature of one or more of items 2 to 19.
(Item 51) A therapy of and/or method for treating a disease, disorder, or condition that can be treated and/or prevented by emricasan in a subject, the method comprising:
   a) a process of reconstituting a composition in a powder form comprising emricasan or a derivative thereof, or a pharmaceutically acceptable salt thereof, or a solvate thereof with a medium for reconstitution; and
   b) a process comprising a process of administering an effective amount of a formulation comprising emricasan prepared by the reconstitution to a subject in need thereof,
   wherein the formulation comprises:
   (i) emricasan or a derivative thereof, or a pharmaceutically acceptable salt thereof, or a solvate thereof;
   (ii) a nonionic surfactant comprising a polyoxyethylene group; and
   (iii) a buffering agent that maintains pH at about 3.0 to about 8.5.
(Item 52) The method of item 51, further comprising a feature of one or more of items 21 to 28.
(Item 53) The method of item 51 or 52, wherein the preparation at the time of use is carried out by using a kit having a feature of any one or more of items 29 to 37.
(Item 54) The method of any of items 51 to 53, wherein the preparation at the time of use is performed by a process having a feature of any one or more of items 38 to 45.
(Item 55) A therapy of and/or formulation for treating a disease, disorder, or condition that can be treated and/or prevented by emricasan, wherein the formulation comprises:
   (i) emricasan or a derivative thereof, or a pharmaceutically acceptable salt thereof, or a solvate thereof;
   (ii) a nonionic surfactant comprising a polyoxyethylene group; and
   (iii) a buffering agent that maintains pH at about 3.0 to about 8.5.
(Item 56) The formulation of item 55, further comprising a feature of one or more of items 2 to 19.

The present disclosure is intended so that one or more of the aforementioned features can be provided not only as the explicitly disclosed combinations, but also as other combinations thereof. Further embodiments and advantages of the present disclosure are recognized by those skilled in the art by reading and understanding the following detailed description as needed.

### [Advantageous Effects of Invention]

The formulation of the present disclosure has a specific condition, and can optimize the soluble property and/or stability of emricasan or a derivative thereof in the formulation.

### [Brief Description of Drawings]

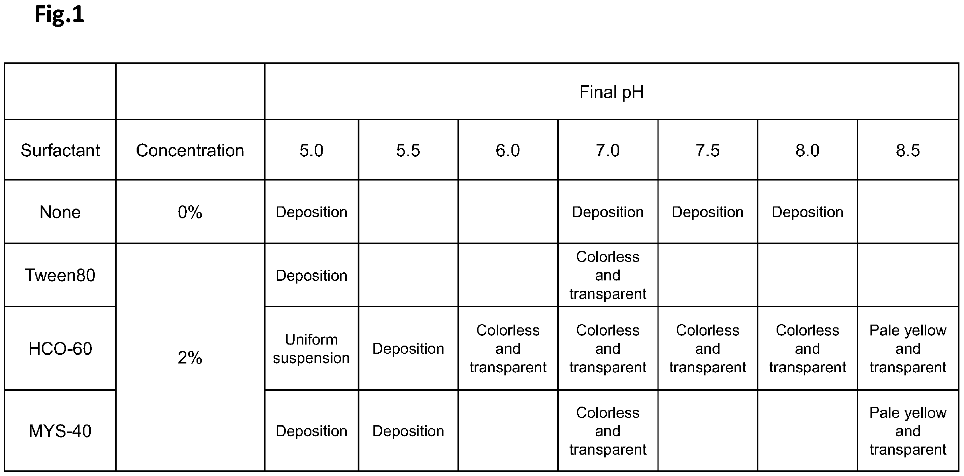

[Figure 1] Figure **1** shows the effect of a surfactant (Tween 80, HCO-60, MYS-40) at various pH on the solubility of emricasan.

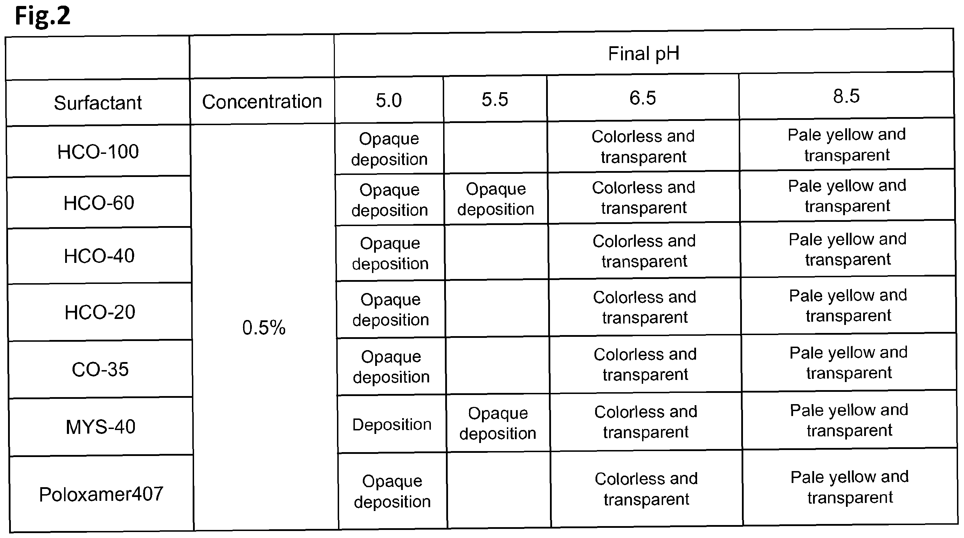

[Figure 2] Figure **2** shows the effect of a surfactant (HCO-100, HCO-60, HCO-40, HCO-20, HCO-20, MYS-40, poloxamer 407) at low pH on the solubility of emricasan.

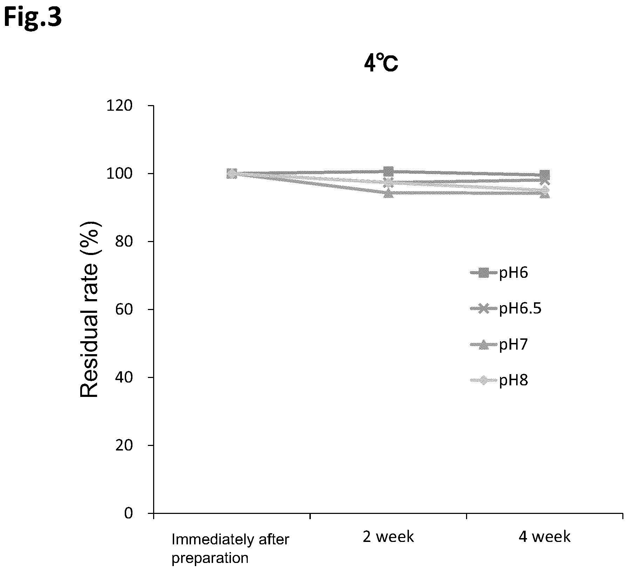

[Figure 3] Figure **3** shows the graph of the stability of emricasan using HCO-60. The vertical axis shows the ratio of the residual amount of emricasan of when the emricasan amount immediately after preparation of an emricasan solution is set to be 100, while the horizontal axis shows the preservation period after preparation of the emricasan solution. Preservation for each case was performed at 4°C, and the emricasan solution for each case was prepared so that pH was pH 6, pH 6.5, pH 7, and pH 8.

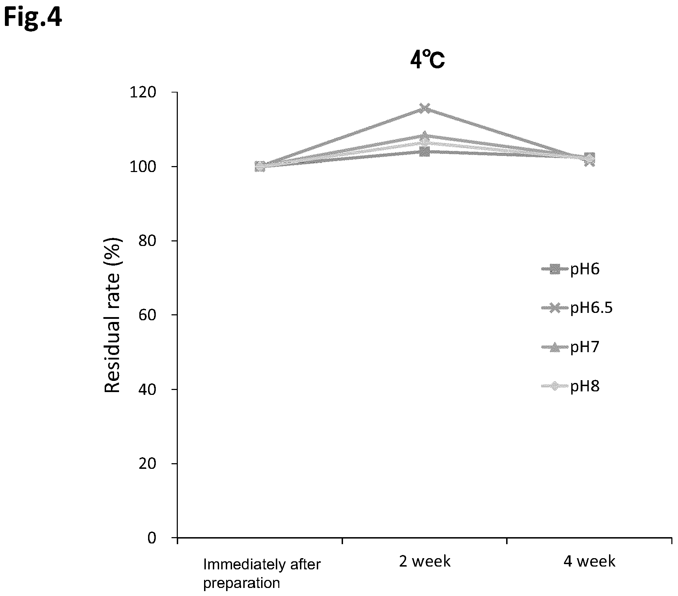

[Figure 4] Figure **4** shows the graph of the stability of emricasan using Tween 80. The vertical axis shows the ratio of the residual amount of emricasan of when the emricasan amount immediately after preparation of an emricasan solution is set to be 100, while the horizontal axis shows the preservation period after preparation of the emricasan solution. Preservation for each case was performed at 4°C, and the emricasan solution for each case was prepared so that pH was pH 6, pH 6.5, pH 7, and pH 8.

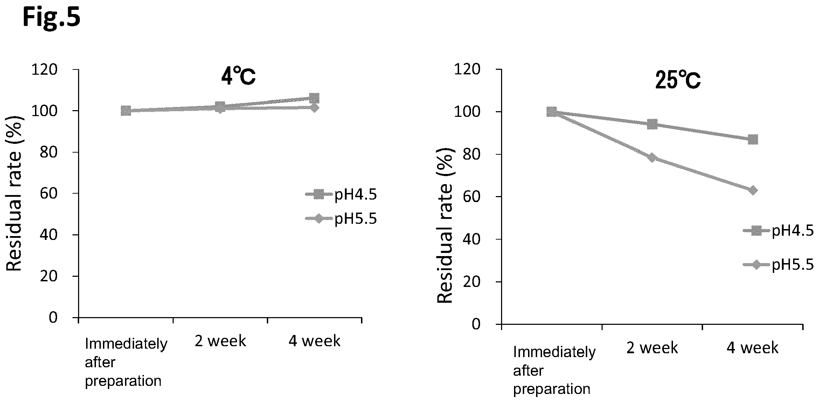

[Figure 5] Figure **5** shows the graph of the stability of emricasan using Tween 80 in an acidic region. The vertical axis shows the ratio of the residual amount of emricasan of when the emricasan amount immediately after preparation of an emricasan solution is set to be 100, while the horizontal axis shows the preservation period after preparation of the emricasan solution. Preservation for each case was performed at 4°C and 25°C, and the emricasan solution for each case was prepared so that pH was pH 4.5 and pH 5.5.

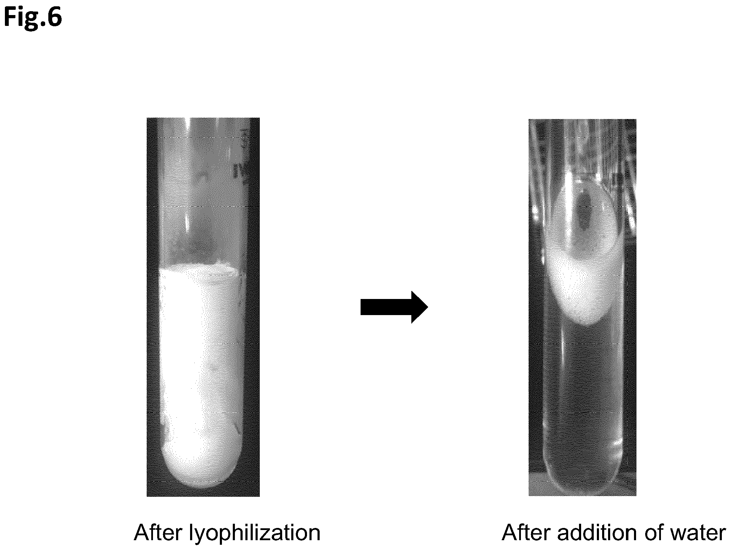

[Figure 6] Figure **6** shows the result of a lyophilization test of an emricasan ophthalmic solution. The photo on the left side shows a lyophilized product of the emricasan ophthalmic solution, while the photo on the right side shows a solution wherein the lyophilized product of the emricasan ophthalmic solution is dissolved with water.

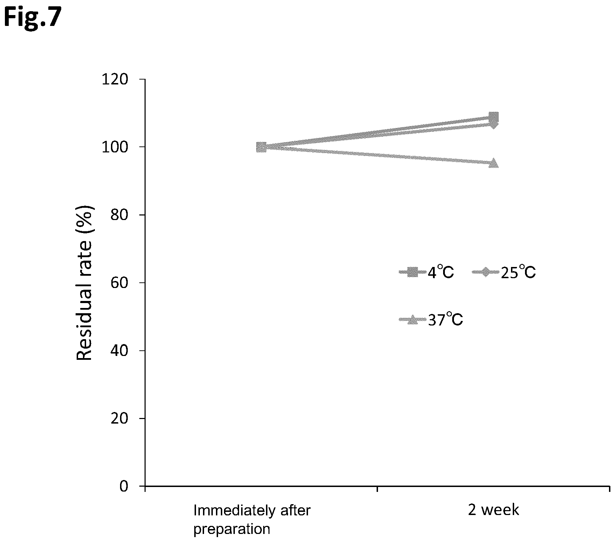

[Figure 7] Figure **7** shows the graph of the stability of lyophilized powder of an emricasan ophthalmic solution. The vertical axis shows the ratio of the residual amount of emricasan of when the emricasan amount immediately after preparation of an emricasan solution is set to be 100, while the horizontal axis shows the preservation period after preparation of the emricasan solution. Preservation for each case was performed at 4°C, 25°C, and 37°C.

### [Description of Embodiments]

The present disclosure is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definition)

As used herein, "about" before a numerical value means ±10% of the numerical value that follows.

As used herein, "caspase" is a general term for endopeptidases that hydrolyze a peptide bond at the C-terminus side of aspartic acid, with cysteine as the center of activity. Caspases are known to have a function in processing cytokines (interleukin 1β and the like), and have involvement in execution of programmed cell death and inflammatory responses. All caspases are translated as an enzyme precursor. Caspases are activated by degradation by another caspase or themselves and function in a form of a cascade. Caspases are assigned a number in the order of discovery. Currently, caspase 1 to caspase 14 are known in mammals. About 10 types of caspases have been discovered in human cells. For example, caspase 1 has a function in inducing inflammation by processing cytokines, caspase 3 is directly involved in the execution of programmed cell death, and caspase 8 is positioned upstream the cascade and is responsible for signaling in programmed cell death.

As used herein, "caspase inhibitor" refers to any agent that inhibits signaling of any caspase. A caspase inhibitor is therefore a compound that can inhibit one or more of a caspase family. Emricasan is a pan caspase inhibitor that can inhibit all caspases.

As used herein, "iFECD" (immortalized Fuchs' endothelial corneal dystrophy) is an abbreviation for immortalized cells in Fuchs' endothelial corneal dystrophy.

As used herein, "HCEC" (human cornel endothelial cells) is an abbreviation for human corneal endothelial cells. In addition, "iHCEC" is an abbreviation for immortalized human corneal endothelial cells.

As used herein, a "derivative" refers to a compound which has a core structure that is the same as or very similar to that of a parent compound but has a chemical modification such as a different functional group or an additional functional group. A derivative has biological activity that is the same as or similar to that of a parent compound.

As used herein, a "pharmaceutically acceptable salt" refers to a salt of the compound of the present disclosure (e.g., acidic salt, basic salt, and the like) which is relatively non-toxic. These salts can be temporarily prepared during the final isolation and purification of a compound, or can be prepared by causing a compound purified by the free base form to individually react with a suitable organic or inorganic acid, and isolating a salt formed in such a manner.

A pharmaceutically acceptable basic salt of the compound of the present disclosure includes, for example: alkali metal salt such as sodium salt or potassium salt; alkaline earth metal salt such as calcium salt or magnesium salt; ammonium salt; aliphatic amine salt such as trimethylamine salt, triethylamine salt, dicyclohexylamine salt, ethanolamine salt, diethanolamine salt, triethanolamine salt, procaine salt, meglumine salt, diethanolamine salt or ethylenediamine salt; aralkylamine salt such as N,N-dibenzylethylenediamine and benethamine salt; heterocyclic aromatic amine salt such as pyridine salt, picoline salt, quinoline salt, or isoquinoline salt; quaternary ammonium salt such as tetramethylammonium salt, tetraethylammonium salt, benzyltrimethylammonium salt, benzyltriethylammonium salt, benzyltributylammonium salt, methyltrioctylammonium salt, or tetrabutylammonium salt; basic amino acid salt such as arginine salt or lysine salt, and the like.

A pharmaceutically acceptable acidic salt of the compound of the present disclosure includes, for example: inorganic acid salt such as hydrochloride salt, sulfuric acid salt, nitric acid salt, phosphoric acid salt, carbonic acid salt, hydrogencarbonate salt, or perchloric acid salt; organic acid salt such as acetic acid salt, propionic acid salt, lactic acid salt, maleic acid salt, fumaric acid salt, tartaric acid salt, malic acid salt, citric acid salt, or ascorbic acid salt; sulfonic acid salt such as methanesulfonic acid salt, isethionic acid salt, benzenesulfonic acid salt, or p-Toluenesulfonic acid salt; acidic amino acid such as aspartic acid salt or glutamic acid salt, and the like.

As used herein, a "solvate" means a solvate of the compound of the present disclosure or a pharmaceutically acceptable salt thereof, and encompasses, for example, a solvate with an organic solvent (e.g., solvate with alcohol (such as ethanol)), hydrate and the like. When a hydrate is formed, the hydrate may be coordinated with any number of water molecules. A hydrate can include monohydrate, dihydrate and the like.

As used herein, a "surfactant" refers to a substance that has a hydrophilic moiety and a hydrophobic moiety in a molecule and has action of dissolving into liquid to significantly lower the surface tension. As used herein, a "nonionic surfactant" refers to a surfactant that is not ionized to be an ion when dissolved into water. A "nonionic surfactant comprising a polyoxyethylene group" includes, but is not limited to, polyoxyethylene hydrogenated castor oil, polyoxyethylene castor oil, polyoxyethylene fatty acid ester, polyoxyethylene sorbitan fatty acid ester, and poloxamer.

As used herein, "polyoxyethylene hydrogenated castor oil" refers to a compound wherein polyoxyethylene is subjected to addition polymerization to hydrogenated (added with hydrogen) castor oil, and has the following structure: 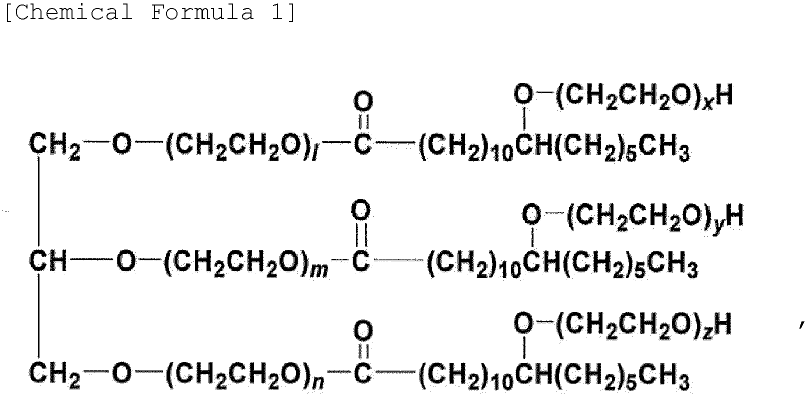 wherein the sum of 1, m, n, x, y, and z is generally, but not limited to, 5 to 120. For example, polyethylene hydrogenated castor oil wherein the sum of 1, m, n, x, y, and z is 20 is called polyethylene hydrogenated castor oil-20 (HCO-20). For example, polyoxyethylene hydrogenated castor oil includes, but is not limited to, HCO-20, HCO-40, HCO-60, HCO-80, and HCO-100.

As used herein, "polyoxyethylene castor oil" refers to a compound wherein polyoxyethylene is subjected to addition polymerization to castor oil which is not hydrogenated (added with hydrogen). For example, polyoxyethylene castor oil includes, but is not limited to, CO-3, CO-10, and CO-35.

As used herein, "polyoxyethylene fatty acid ester" refers to a compound having the following structure: RCOO-(CH₂CH₂O)ₙH, wherein, but not limited to: R is an alkyl group generally having 12 to 18 carbons or an alkenyl group generally having 12 to 18 carbons; the alkyl group or alkenyl group may be a strain chain or a branched chain; and n is generally 10 to 70. Specifically, examples include, but are not limited to, polyethylene glycol monolaurate 10 (MYL-10; n = 10), polyethylene glycol monostearate 10 (MYS-10; n = 10), polyethylene glycol monostearate 25 (MYS-25; n = 25), polyethylene glycol monostearate 40 (MYS-40; n = 40), and polyethylene glycol monostearate 55 (MYS-55; n = 55).

As used herein, "polyoxyethylene sorbitan fatty acid ester" refers to a compound having the following structure: 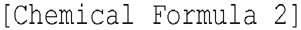 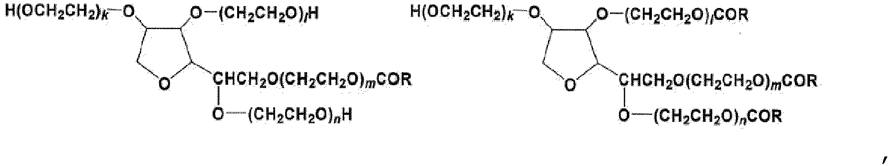 wherein, but not limited to: R is an alkyl group generally having 12 to 18 carbons, an alkenyl group generally having 12 to 18 carbons, or an alkynyl group generally having 12 to 18 carbons; the alkyl group, alkenyl group and alkynyl group may be a straight chain or a branched chain; and the sum of k, 1, m, and n is generally 20. Specifically, examples include, but are not limited to, polysorbate 80 (polyoxyethylene sorbitan monooleate) (also called Tween 80), polysorbate 60 (polyoxyethylene sorbitan monostearate) (also called Tween 60), polysorbate 40 (polyoxyethylene sorbitan monopalmitate) (also called Tween 40), polyoxyethylene monolaurate, polyethylene sorbitan trioleate, and polysorbate 65 (polyoxyethylene sorbitan tristearate) (also called Tween 65).

As used herein, "poloxamer" refers to a polyoxyethylene-polyoxypropylene block copolymer generally having a molecular weight within the range of about 2000 to about 15,000 daltons and having the following general formula:

HO(C₂H₄O)ₐ(C₃H₆O)_{b}(C₂H₄O)ₐH,

wherein, depending on a particular grade: a is from about 10 to about 150 and represents a block of repeating units of polyoxyethylene (hereinafter, referred to as the PEO moiety); and b is from about 20 to about 60 and represents a block of repeating units of polyoxypropylene (hereinafter, referred to as the PPO moiety). Specific examples of poloxamer include poloxamer 124, poloxamer 188 (PLURONIC F68), poloxamer 237 (PLURONIC F87), poloxamer 338 (PLURONIC F108), poloxamer 407 (PLURONIC F127) shown in Table 1, and a mixture thereof.

**[Table 1]**

| **Poloxamer** | **a** | ***b*** |
|---|---|---|
| **124** | **12** | **20** |
| **188** | **80** | **27** |
| **237** | **64** | **37** |
| **338** | **141** | **44** |
| **407** | **101** | **56** |

As used herein, a "buffering agent" refers to one type or more types of compounds that, when added to a certain solution, exhibit an ability of causing little or no change in a predetermined pH of the solution, or an aqueous solution in which said compounds are dissolved. For example, a buffering agent includes, but is not limited to, phosphoric acid buffering agent, citric acid buffering agent, boric acid buffering agent, carbonic acid buffering agent, acetic acid buffering agent, tartaric acid buffering agent, aminocarboxylic acid buffering agent, trometamol, and the like.

As used herein, a "corneal endothelial condition, disorder, or disease" refers to any condition, disorder, or disease caused to a corneal endothelium. For example, a corneal endothelial condition, disorder, or disease includes, but is not limited to, Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), and idiopathic corneal endothelial disorder, and the like.

As used herein, "preparation at the time of use" refers to dissolving lyophilized powder comprising emricasan into a medium for reconstitution (e.g., purified water, saline, or solution for reconstitution comprising other components or the like) and preparing a formulation suitable for administration comprising emricasan immediately before administering emricasan to a subject. Further, "preparation at the time of use" may also refer to preparation immediately before each administration of emricasan, and may also refer to preparation of a formulation immediately before the first administration of when the formulation is preserved for a short period of time (e.g., a few days, a few weeks) at room temperature, in a cool place, or under refrigeration after prepared to then be used. Thus, preparation at the time of use is intended for both using up a formulation every time it is administered, and preserving a prepared formulation for a short period of time and repeatedly using it. As used herein, a formulation which is prepared at the time of use is called formulation prepared at the time of use.

As used herein, a "subject" refers to a target of administration of the formulation of the present disclosure. Examples of subjects include mammals (e.g., human, mouse, rat, hamster, rabbit, cat, dog, cow, horse, sheep, monkey and the like), while primates are preferable, and humans are particularly preferable.

As used herein, "reconstitution" refers to adding water or solution to dry powder such as lyophilized powder, thereby restoring a dry state to a liquid state.

As used herein, a "medium for reconstitution" refers to a solution that is added to dry powder such as lyophilized powder to render a liquid state. A medium for reconstitution includes purified water (e.g., sterilized purified water), saline, buffering agent, or any pharmaceutically acceptable aseptic solution for reconstitution, or any combination thereof. In addition to the above, a medium for reconstitution that can be used in the present disclosure includes a buffering agent, a surfactant, an antiseptic and the like. An antiseptic can be comprised in order to prevent secondary pollution. For example, a surfactant that can be comprised as a medium for reconstitution can include, but is not limited to, a nonionic surfactant comprising a polyoxyethylene group. Any surfactant disclosed herein can be utilized, and any buffering agent disclosed herein can be used as a buffering agent. Preferably, for example, a buffering agent that maintains pH at about 3.0 to about 8.5 can be used. For example, an antiseptic includes, but is not limited to, an antiseptic that is generally used for a formulation (e.g., ophthalmic formulation) such as benzalkonium chloride, parabens, sorbic acid salt, benzyl alcohol, or chlorhexidine gluconate. A medium for reconstitution is also called reconstitution solution. Alternatively, these terms may be called medium or solution for short.

As used herein, a "kit" refers to a unit providing portions to be provided (e.g., test drug, diagnosis drug, therapeutic drug, antibody, label, manual, and the like), generally in two or more separate sections. This form of a kit is preferred when intending to provide a composition that should not be provided in a mixed state but is preferably mixed immediately before use for stability reasons or the like. Alternatively, this form of a kit is preferred when it is necessary to dissolve lyophilized powder with a suitable solvent immediately before use for preparation at the time of use when providing a compound which is unstable in a solution state. Such a kit advantageously comprises instructions or a manual preferably describing how the provided portions (e.g., test drug, diagnosis drug, therapeutic drug) should be used or how a reagent should be handled.

### (Disclosure of preferred embodiments)

The preferred embodiments of the present invention are described hereinafter. It should be understood that the embodiments are exemplification of the present disclosure, so that the scope of the present disclosure is not limited to such preferred embodiments. It should be understood that those skilled in the art can refer to the following preferred embodiments to readily make modifications or changes within the scope of the present disclosure. These embodiments can be appropriately combined with any embodiment by those skilled in the art.

### (Formulation)

In one embodiment, the present disclosure provides a formulation comprising: (i) emricasan or a derivative thereof, or a pharmaceutically acceptable salt thereof, or a solvate thereof; (ii) a nonionic surfactant comprising a polyoxyethylene group; and (iii) a buffering agent that maintains pH at about 4.5 to about 8.5.

In some embodiments, a buffering agent can be a buffering agent that maintains pH at about 3.0 to about 8.5, about 3.5 to about 8.0, about 3.5 to about 7.5, about 3.5 to about 7.0, about 3.5 to about 6.5, about 3.5 to about 6.0, about 4.0 to about 7.0, about 4.0 to about 6.5, about 4.0 to about 6.0, about 4.0 to about 5.5, about 4.0 to about 5.0, about 4.5 to about 5.0, about 4.5 to about 5.5, about 4.5 to about 6.0, about 4.5 to about 6.5, about 4.5 to about 7.0, about 4.5 to 7.5, about 4.5 to 8.0, about 5.0 to about 5.5, about 5.0 to about 6.0, about 5.0 to about 6.5, about 5.0 to about 7.0, about 5.0 to about 7.5, about 5.0 to about 7.5, about 5.0 to about 8.0, about 5.0 to about 8.5, about 5.5 to about 6.0, about 5.5 to about 6.5, about 5.5 to about 7.0, about 5.5 to about 7.5, about 5.5 to about 8.0, about 5.5 to about 8.5, about 6.0 to about 6.5, about 6.0 to about 7.0, about 6.0 to about 7.5, about 6.0 to about 8.0, about 6.0 to about 8.5, about 6.5 to about 7.0, about 6.5 to about 7.5, about 6.5 to about 8.0, about 6.5 to about 8.5, about 7.0 to about 7.5, about 7.0 to about 8.0, about 7.0 to about 8.5, about 7.5 to about 8.0, about 7.5 to about 8.5, or about 8.0 to about 8.5.

In a particular embodiment, a buffering agent can be a buffering agent that maintains pH at about 6.0 to about 8.0, about 6.0 to about 7.0, or about 6.0 to about 6.5. In another preferred embodiment, a buffering agent is a buffering agent that maintains pH at about 3.5 to about 8.0, about 4.0 to about 7.0, about 4.0 to about 6.0, or, about 4.5 to about 7.0. In a specific embodiment, a buffering agent can be a buffer that maintains pH at about 3.0, about 3.5, about 4.0, about 4.5, about 5.0, about 5.5, about 6.0, about 6.5, about 7.0, about 7.5, about 8.0, or about 8.5.

Thus, in some embodiments, the pH of the formulation of the present disclosure can be about 3.0 to about 8.5, about 3.5 to about 8.0, about 3.5 to about 7.5, about 3.5 to about 7.0, about 3.5 to about 6.5, about 3.5 to about 6.0, about 4.0 to about 7.0, about 4.0 to about 6.5, about 4.0 to about 6.0, about 4.0 to about 5.5, about 4.0 to about 5.0, about 4.5 to about 5.0, about 4.5 to about 5.5, about 4.5 to about 6.0, about 4.5 to about 6.5, about 4.5 to about 7.0, about 4.5 to 7.5, about 4.5 to 8.0, about 5.0 to about 5.5, about 5.0 to about 6.0, about 5.0 to about 6.5, about 5.0 to about 7.0, about 5.0 to about 7.5, about 5.0 to about 7.5, about 5.0 to about 8.0, about 5.0 to about 8.5, about 5.5 to about 6.0, about 5.5 to about 6.5, about 5.5 to about 7.0, about 5.5 to about 7.5, about 5.5 to about 8.0, about 5.5 to about 8.5, about 6.0 to about 6.5, about 6.0 to about 7.0, about 6.0 to about 7.5, about 6.0 to about 8.0, about 6.0 to about 8.5, about 6.5 to about 7.0, about 6.5 to about 7.5, about 6.5 to about 8.0, about 6.5 to about 8.5, about 7.0 to about 7.5, about 7.0 to about 8.0, about 7.0 to about 8.5, about 7.5 to about 8.0, about 7.5 to about 8.5, or about 8.0 to about 8.5. In a specific embodiment, the pH of the formulation of the present disclosure can be about 3.0, about 3.5, about 4.0, about 4.5, about 5.0, about 5.5, about 6.0, about 6.5, about 7.0, about 7.5, about 8.0, or about 8.5. Those skilled in the art can appropriately adjust the pH of the formulation of the present disclosure by using the above buffering agent.

As long as the final pH of a formulation is encompassed by the above range of pH, different buffering agents may be used in combination, a buffering agent (if any) comprised in a solution upon lyophilization may differ from a buffering agent (if any) comprised in a solvent for reconstitution, or multiple different buffering agents may be comprised in a solution upon lyophilization or a solvent for reconstitution.

Nearly neutral pH (about 4.0 to about 8.0, about 5.0 to about 7.0, preferably about 6.0) is preferred in consideration of the solubility of emricasan, while pH on the acidic side (about 3.5 to about 5.5, preferably about 4.5) is preferred in consideration of the stability of emricasan. Emricasan is preferably prepared through lyophilization in consideration of the stability of emricasan. Although emricasan has a lower solubility at pH on the acidic side as compared to nearly neutral pH, at least about 0.1 % by weight of emricasan, which is beyond an effective concentration (i.e., 100 fold or greater with respect to the concentration 10 µmol/L, which exhibited pharmacological activity in an in vitro test using immortalized cells in Fuchs' endothelial corneal dystrophy (iFECD) (International Publication No. WO 2017/110094)), dissolves.

From the viewpoint of stability, a formulation prepared at the time of use may be preserved for a short period of time at pH on the acidic side (about 3.5 to about 5.5, preferably about 4.5), or may be adjusted to pH on the neutral side (about 4.0 to about 8.0, about 5.0 to about 7.0, preferably about 6.0) upon administration.

In some embodiment, a surfactant can be present in the formulation of the present disclosure at about 0.05% by weight to about 5% by weight, about 0.1% by weight to about 5% by weight, preferably, about 0.1% by weight to about 4% by weight, and more preferably, about 0.1% by weight to about 2% by weight.

In some embodiment, a nonionic surfactant comprising a polyoxyethylene group includes, but is not limited to, polyoxyethylene hydrogenated castor oil, polyoxyethylene castor oil, polyoxyethylene fatty acid ester (e.g., polyethylene glycol monostearate), polyoxyethylene sorbitan fatty acid ester, and polyoxyethylene-polyoxypropylene copolymer (poloxamer).

For example, polyoxyethylene hydrogenated castor oil includes, but is not limited to, HCO-20, HCO-40, HCO-60, HCO-80, and HCO-100. In a preferred embodiment, polyoxyethylene hydrogenated castor oil is HCO-60.

For example, polyoxyethylene castor oil includes, but is not limited to, CO-3, CO-10, and CO-35. In a preferred embodiment, polyoxyethylene castor oil is CO-35.

Polyoxyethylene fatty acid ester includes, but is not limited to, polyethylene glycol monolaurate 10 (MYL-10), polyethylene glycol monostearate 10 (MYS-10), polyethylene glycol monostearate 25 (MYS-25), polyethylene glycol monostearate 40 (MYS-40), and polyethylene glycol monostearate 55 (MYS-55). In a preferred embodiment, polyoxyethylene fatty acid ester is MYS-40.

Polyoxyethylene sorbitan fatty acid ester includes, but is not limited to, polysorbate 80 (Tween 80), polysorbate 60 (Tween 60), polysorbate 40 (Tween 40), polyoxyethylene monolaurate, polyethylene sorbitan trioleate, and polysorbate 65 (Tween 65). In a preferred embodiment, polyoxyethylene sorbitan fatty acid ester is polysorbate 80 (Tween 80).

Polyoxyethylene-polyoxypropylene copolymer (poloxamer) includes, but is not limited to, poloxamer 124, poloxamer 188, poloxamer 237, poloxamer 338, poloxamer 407. In a preferred embodiment, polyoxyethylene-polyoxypropylene copolymer (poloxamer) is poloxamer 407.

In some embodiments, emricasan or a derivative thereof, or a pharmaceutically acceptable salt thereof, or a solvate thereof can be present in the formulation at about 0.05% by weight to about 5% by weight, about 0.1% by weight to about 2% by weight, preferably, about 0.1% by weight to about 1% by weight, and more preferably, about 0.1% by weight to about 0.5% by weight.

In one preferred embodiment, the present disclosure provides a formulation comprising emricasan, wherein the formulation comprises:
(i) emricasan that is present in the formulation at about 0.1% by weight to about 1% by weight; (ii) a surfactant selected from the group consisting of HCO-20, HCO-40, HCO-60, HCO-80, HCO-100, CO-35, MYS-40, Tween 80, and poloxamer 407, the surfactant being present in the formulation at about 0.1% by weight to about 4% by weight; and (iii) a buffering agent that maintains pH at about 3.0 to about 8.0, and preferably about 4.0 to about 7.0.

In another preferred embodiment, the present disclosure provides a formulation comprising emricasan, wherein the formulation comprises: (i) emricasan that is present in the formulation at about 0.1% by weight to about 1% by weight; (ii) HCO-60 that is present in the formulation at about 0.1% by weight to about 4% by weight; and (iii) a buffering agent that maintains pH at about 4.0 to about 7.0.

In another preferred embodiment, provided is a formulation comprising emricasan, wherein the formulation comprises: (i) emricasan that is present in the formulation at about 0.1% by weight to about 1% by weight; (ii) Tween 80 that is present in the formulation at about 0.1% by weight to about 4% by weight; and (iii) a buffering agent that maintains pH at about 4.0 to about 7.0.

The formulation of the present disclosure may further comprise an agent such as an antiseptic, a stabilizer, an isotonicifier, a pH adjuster, or a lyophilization adjuvant which is generally used for a formulation (e.g., ophthalmic formulation such as an eye drop) as needed.

In some embodiments, the formulation of the present disclosure is an ophthalmic formulation. In a particular embodiment, the formulation of the present disclosure can be a formulation for treating or preventing a corneal endothelial condition, disorder, or disease in a corneal endothelium. In particular, emricasan, which is comprised in the formulation of the present disclosure, is very effective for a corneal endothelial condition, disorder, or disease due to transforming growth factor-β (TGF-β) (International Publication No. WO 2017/110094, which is incorporated herein by reference). Examples include, but are not limited to, Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), and idiopathic corneal endothelial disorder in a corneal endothelium.

In some embodiments, examples of utilization methods of the formulation of the present disclosure include, but are not limited to, an eye drop, as well as administration methods such as injection into the anterior chamber, impregnation into a controlled-release agent, subconjunctival injection, and systemic administration (oral administration and intravenous injection).

The form of the formulation of the present disclosure may be a form of any of powder, solid, and liquid, but is preferably a form of liquid, particularly a form of aqueous solution.

The formulation of the present disclosure may further comprise a saccharide such as mannitol, sucrose, and trehalose as needed.

In another embodiment, the present invention provides a therapy of and/or formulation for treating a disease, disorder, or condition that can be treated and/or prevented by emricasan, wherein the formulation comprises: (i) emricasan or a derivative thereof, or a pharmaceutically acceptable salt thereof, or a solvate thereof; (ii) a nonionic surfactant comprising a polyoxyethylene group; and (iii) a buffering agent that maintains pH at about 3.0 to about 8.5.

### (Preservation method)

In the case of long-term preservation, the formulation of the present disclosure is preferably preserved in a dehydrated state (e.g., powder form, particularly lyophilized powder form). The formulation of the present disclosure can be preserved at about 4°C to about 25°C for the stability of emricasan. In a particular embodiment, when the formulation is preserved as a solution, it is preferable to preserve the formulation at a low temperature (about 4°C), and it is more preferable to preserve the formulation while maintaining pH of the solution on the acidic side (pH about 3.0 to about 5.5).

Thus, in another embodiment of the present disclosure, the present disclosure provides a method for preserving emricasan, characterized in that emricasan is preserved in a dehydrated state (e.g., powder form, particularly lyophilized powder form). In yet another embodiment, the present disclosure provides a method for preserving emricasan in a solution state, characterized in that the solution is preserved so as to maintain a low temperature (about 4°C) and pH on the acidic side (pH about 4.5 to about 5.5). In a preferred embodiment, the formulation in a solution state is preserved to maintain about 4°C and pH about 4.5.

### (Composition for preparation at the time of use)

It was elucidated that emricasan is very unstable in a solution but very stable in a lyophilized state. Further, it was elucidated that it is possible to relatively stabilize emricasan under a specific condition even after reconstituting the lyophilized powder of emricasan. In this manner, it is possible to stably provide a formulation of emricasan by the composition for preparation at the time of use of the present disclosure which comprises lyophilized powder of emricasan. Thus, in another embodiment of the present disclosure, the present disclosure provides a composition comprising emricasan or a derivative thereof, or a pharmaceutically acceptable salt thereof, or a solvate thereof in a powder form (e.g., lyophilized powder form), characterized in being dissolved into a medium for reconstitution (e.g., purified water, saline, buffer, or any combination thereof) to be prepared. The composition of the present disclosure may further comprise an agent such as an antiseptic, a stabilizer, an isotonicifier, a pH adjuster, or a lyophilization adjuvant as needed.

In a preferred embodiment, the composition of the present disclosure comprises: (i) emricasan or a derivative thereof, or a pharmaceutically acceptable salt thereof, or a solvate thereof; (ii) a nonionic surfactant comprising a polyoxyethylene group; and (iii) a buffering agent that maintains pH at about 3.0 to about 8.5. In a preferred embodiment, the medium for reconstitution which is used in the present disclosure is, for example, purified water, saline, buffering agent, or any combination thereof. The composition of the present disclosure is dissolved into these media for reconstitution to be prepared.

In one embodiment, a medium for reconstitution can comprise a nonionic surfactant comprising a polyoxyethylene group. In another embodiment, a medium for reconstitution can comprise a buffering agent that maintains pH at about 3.0 to about 8.5. Preferred pH as a medium for reconstitution can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like). Since pH closer to tear fluid can be preferred upon reconstitution, pH that can be employed may be nearly neutral (e.g., pH about 6.0 to about 8.0).

The pH upon lyophilization (solution immediately before lyophilization) may differ from the pH of a medium for reconstitution. The pH upon lyophilization may be on the acidic side (pH about 3.5 to about 5.5, preferably about 4.5) from the viewpoint of stability. The pH upon reconstitution may be neutral pH (about 4.0 to about 8.0, preferably about 6.0) which is suitable for solubility and/or administration.

A solvent for reconstitution may further comprise an agent such as an antiseptic, a stabilizer, an isotonicifier, or a pH adjuster as needed.

The medium for reconstitution which is used in the present disclosure may further comprise a saccharide such as mannitol, sucrose, and trehalose as needed.

### (Kit)

In another embodiment, the present disclosure provides a kit for preparing the above-described formulation at the time of use, wherein the kit comprises: (i) powder (e.g., lyophilized powder) of emricasan or a derivative thereof, or a pharmaceutically acceptable salt thereof, or a solvate thereof; (ii) a nonionic surfactant comprising a polyoxyethylene group as needed; and (iii) a buffering agent that maintains pH at about 3.0 to about 8.5 as needed. The pH that can be employed in this case can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like).

In some embodiments, the kit of the present disclosure may or may not comprise a nonionic surfactant comprising a polyoxyethylene group and/or a buffering agent. A user of the kit of the present disclosure may obtain a nonionic surfactant comprising a polyoxyethylene group and/or a buffering agent from another provider. In some embodiments, the kit of the present disclosure may comprise a manual for use.

In yet another embodiment, provided is a kit for preparing the above-described formulation at the time of use, wherein the kit comprises: (i) powder (e.g., lyophilized powder) of emricasan or a derivative thereof, or a pharmaceutically acceptable salt thereof, or a solvate thereof; (ii) a medium for reconstitution which comprises a nonionic surfactant comprising a polyoxyethylene group and a buffering agent that maintains pH at about 3.0 to about 8.5 as needed. The pH that can be employed in this case can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like). Since pH closer to tear fluid can be preferred upon reconstitution, the pH that can be employed may be nearly neutral (e.g., pH about 6.0 to about 8.0).

### (Method for preparation at the time of use)

In another embodiment, the present disclosure provides a method for preparing the above-described formulation at the time of use, comprising: (i) providing powder which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5; and (ii) reconstituting the powder with a medium for reconstitution. In some embodiments, powder can be powder obtained by lyophilizing a solution which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5.

In yet another embodiment, the present disclosure provides a method for preparing the above-described formulation at the time of use, comprising: (i) providing powder (e.g., lyophilized powder) of emricasan or a derivative thereof, or a pharmaceutically acceptable salt thereof, or a solvate thereof; and (ii) reconstituting the powder with a medium for reconstitution which comprises a nonionic surfactant comprising a polyoxyethylene group and a buffering agent that maintains pH at about 3.0 to about 8.5. The pH that can be employed in this case can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like).

The pH upon lyophilization (solution immediately before lyophilization) may differ from the pH of a medium for reconstitution. The pH upon lyophilization may be on the acidic side (pH about 3.5 to about 5.5, preferably about 4.5) from the viewpoint of stability. The pH upon reconstitution may be neutral pH (about 4.0 to about 8.0, preferably about 6.0) which is suitable for solubility and/or administration.

### (Solution for reconstitution)

In another embodiment, the present disclosure provides a solution for reconstituting lyophilized powder of emricasan or a derivative thereof, or a pharmaceutically acceptable salt thereof, or a solvate thereof, wherein the solution comprises: (i) a solvent; (ii) a nonionic surfactant comprising a polyoxyethylene group; and (iii) a buffering agent that maintains pH at about 3.0 to about 8.5.

In some embodiments, a solvent is selected from the group consisting of purified water, saline, or any combination thereof. The pH that can be employed in this case can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like). Since pH closer to tear fluid can be preferred upon reconstitution, the pH that can be employed may be nearly neutral (e.g., pH about 6.0 to about 8.0).

The pH upon lyophilization (solution immediately before lyophilization) may differ from the pH of a medium for reconstitution. The pH upon lyophilization may be on the acidic side (pH about 3.5 to about 5.5, preferably about 4.5) from the viewpoint of stability. The pH upon reconstitution may be neutral pH (about 4.0 to about 8.0, preferably about 6.0) which is suitable for solubility and/or administration.

### (Solution for dissolution)

In another embodiment, the present disclosure provides a solution for dissolving a crystal or powder thereof of emricasan or a derivative thereof, or a pharmaceutically acceptable salt thereof, or a solvate thereof, wherein the solution comprises: (i) a solvent; (ii) a nonionic surfactant comprising a polyoxyethylene group; and (iii) a buffering agent that maintains pH at about 3.0 to about 8.5. The pH that can be employed in this case can be the same as a preferred numerical value of pH of a buffering agent described in other parts of the present specification, and can be, for example, pH about 4.0 to about 7.0 (e.g., about 4.0, about 4.5, about 5.0, about 5.5, about 6.0, about 6.5, about 7.0 or the like). Since pH closer to tear fluid can be preferred upon reconstitution, the pH that can be employed may be nearly neutral (e.g., pH about 6.0 to about 8.0) .

The pH upon lyophilization (solution immediately before lyophilization) may differ from the pH of a solution for dissolution. The pH upon lyophilization may be on the acidic side (pH about 3.5 to about 5.5, preferably about 4.5) from the viewpoint of stability. The pH upon dissolution may be neutral pH (about 4.0 to about 8.0, preferably about 6.0) which is suitable for solubility and/or administration.

In some embodiments, a solvent is selected from the group consisting of purified water, saline, or any combination thereof.

The solution of the present disclosure may further comprise an agent such as an antiseptic, a stabilizer, an isotonicifier, or a pH adjuster as needed.

### (Therapy and prophylaxis)

In other embodiments, provided can be a method for treating or preventing a corneal endothelial condition, disorder, or disease, comprising administering a therapeutically effective amount of the formulation of the present disclosure to a subject. One or more embodiments and/or features described above can be appropriately employed in the embodiments of the present disclosure.

For example, in one embodiment, the present invention provides a therapy of and/or method for treating a disease, disorder, or condition that can be treated and/or prevented by emricasan in a subject, comprising a process of administering an effective amount of a formulation comprising emricasan to the subject in need thereof. In this method, the formulation comprises: (i) emricasan or a derivative thereof, or a pharmaceutically acceptable salt thereof, or a solvate thereof; (ii) a nonionic surfactant comprising a polyoxyethylene group; and (iii) a buffering agent that maintains pH at about 3.0 to about 8.5.

As used herein, "a disease, disorder, or condition that can be treated and/or prevented by emricasan" can include any disease, disorder, or condition that can be treated and/or prevented by emricasan. Examples thereof can include cancer, Fuchs' endothelial corneal dystrophy and the like. In this method, any feature explained in any one or more of the sections of (Formulation), (Preservation method), (Composition for preparation at the time of use), (Kit), (Method for preparation at the time of use), (Solution for reconstitution), and (Solution for dissolution) can be applied alone or in combination.

In another embodiment, the present disclosure provides a therapy of and/or method for treating a disease, disorder, or condition that can be treated and/or prevented by emricasan in a subject. This method comprises: a) a process of reconstituting a composition in a powder form comprising emricasan or a derivative thereof, or a pharmaceutically acceptable salt thereof, or a solvate thereof with a medium for reconstitution; and b) a process comprising a process of administering an effective amount of a formulation comprising emricasan prepared by the reconstitution to a subject in need thereof, wherein the formulation comprises: (i) emricasan or a derivative thereof, or a pharmaceutically acceptable salt thereof, or a solvate thereof; (ii) a nonionic surfactant comprising a polyoxyethylene group; and (iii) a buffering agent that maintains pH at about 3.0 to about 8.5. In this method as well, any feature explained in any one or more of the sections of (Formulation), (Preservation method), (Composition for preparation at the time of use), (Kit), (Method for preparation at the time of use), (Solution for reconstitution), and (Solution for dissolution) can be applied alone or in combination.

In one embodiment, the method of the present disclosure may be carried out by using a kit provided in the present disclosure.

In one embodiment, the formulation of the present disclosure may or may not be prepared at the time of use.

The present disclosure has been explained while showing preferred embodiments to facilitate understanding. The present disclosure is explained hereinafter based on Examples. The above explanation and the following Examples are not provided to limit the present disclosure, but for the sole purpose of exemplification. Thus, the scope of the present disclosure is not limited to the embodiments and Examples that are specifically disclosed herein and is limited only by the scope of claims.

### [Examples]

The present disclosure is more specifically explained hereinafter based on the Examples. It is understood that the specifically shown reagents as well as those available from Sigma-Aldrich, BASF Japan Ltd., or the like can be used as the various reagents used in the Examples.

### (Example 1: Effect of a surfactant and a pH value on solubility of emricasan)

Emricasan is poorly soluble and cannot be dissolved into saline, phosphate buffer, purified water or the like at a concentration for use in an eye drop. Thus, it was tested whether it is possible to dissolve emricasan by using various types of surfactants.

### (Materials and Methods)

In this example, a target dissolution concentration of emricasan was set to be 10 mmol/L, which is 1000 fold with respect to a concentration of 10 µmol/L that exhibited pharmacological activity in an in vitro test using immortalized cells in Fuchs' endothelial corneal dystrophy (iFECD) (International Publication No. WO 2017/110094)). 28 mg, which is the amount for the target concentration, of emricasan powder (Chemscene) was accurately measured with an electronic balance (AUW220D, SHIMADZU) and transferred to a glass test tube. 100 µl of 99% ethanol was added thereto to dissolve the emricasan powder. The solution thereof was sufficiently stirred with a vortex mixer in order to completely dissolve the emricasan powder. The solution thereof was then added with each of polysorbate 80 (Tween 80, NIKKOL), polyoxyethylene hydrogenated castor oil 60 (HCO-60, NIKKOL), and polyethylene glycol monostearate 40 (MYS-40, NIKKOL), which are nonionic surfactants comprising a polyoxyethylene group, so that the final concentration was 2%, followed by sufficient stirring. Phosphate buffered saline (PBS) was added to this solution so that the total amount was 5 ml, and the solution was sufficiently stirred and solubility was visually observed.

### (Results)

Figure 1 shows the results. Since emricasan is an acidic compound, the more the pH value of the solution approaches the basic side, the higher the solubility becomes. Specifically, in the case of an acidic substance, when pH becomes basic, an ionic form concentration increases while a molecular form concentration decreases, which results in higher solubility. Under a condition wherein a surfactant was not used, deposition occurred and dissolution was not observed under the condition of pH value of 7.0 to 8.0. When Tween 80, HCO-60, or MYS-40 was used as a nonionic surfactant comprising a polyoxyethylene group, dissolution of all of the emricasan was observed under the condition of pH 7. Further, under the condition of pH 5.0, emricasan was slightly dissolved by using a surfactant, but deposition occurred and complete dissolution was not observed. Meanwhile, when HCO-60 was used, a suspended state was maintained, and better results as compared to other surfactants were confirmed. When 10 mmol/L was set as a target dissolution concentration, the minimum pH value of a solvent in which dissolution was visually observed was pH 6.0. Thus, in order to achieve the target dissolution concentration in this example, use of a nonionic surfactant comprising a polyoxyethylene group having a final concentration of 2% enabled emricasan to be dissolved at the final pH of 6.0 or greater.

### (Example 2: Effect of various surfactants at a low pH value on solubility of emricasan)

Since it is expected that emricasan would be degraded under a basic condition, solubility at pH lower than pH 7.0 was tested.

### (Materials and Methods)

In this example, a target dissolution concentration of emricasan was set to be 10 mmol/L in the same manner as Example 1. 28 mg of emricasan was accurately measured with an electronic balance (AUW220D, SHIMADZU) and transferred to a glass test tube. 100 µl of 99% ethanol was added thereto to dissolve emricasan. The solution thereof was sufficiently stirred with a vortex mixer in order to completely dissolve emricasan. The solution thereof was then added with polyoxyethylene hydrogenated castor oil 100 (HCO-100, NIKKOL), polyoxyethylene hydrogenated castor oil 60 (HCO-60, NIKKOL), polyoxyethylene hydrogenated castor oil 40 (HCO-40, NIKKOL), polyoxyethylene hydrogenated castor oil 20 (HCO-20, NIKKOL), polyoxyethylene castor oil 35 (CO-35:, NIKKOL), polyethylene glycol monostearate 40 (MYS-40, NIKKOL), and polyoxyethylene polyoxypropylene glycol 407 (poloxamer 407, NIKKOL), which are nonionic surfactants comprising a polyoxyethylene group, so that the final concentration was 0.5%, followed by sufficient stirring. Phosphate buffered saline (PBS) was added to this solution so that the total amount was 5 ml, and the solution was sufficiently stirred and solubility was visually observed.

### (Results)

Figure **2** shows the results. Among the surfactants that were used in this example, the solubility of emricasan exhibited the same tendency. When the final concentration of a surfactant was 0.5%, dissolution was observed at the final pH of 6.5 or greater. Thus, it was found that the solubility of emricasan is subject to pH.

### (Example 3: Stability of emricasan using HCO-60)

In Examples 1 and 2, it was found that emricasan is solubilized within a specific pH range by using a nonionic surfactant comprising a polyoxyethylene group. In this example, the stability of emricasan in a solution was tested.

### (Materials and Methods)

Stability of when HCO-60 was used as a surfactant to dissolve emricasan to a final concentration of 0.5% was tested. 500 mg of emricasan was accurately measured with an electronic balance (AUW220D, SHIMADZU) and transferred to a glass test tube. 2 ml of 99% ethanol was added thereto to dissolve emricasan. The solution thereof was sufficiently stirred with a vortex mixer in order to completely dissolve emricasan. The solution thereof was then added with polyoxyethylene hydrogenated castor oil 60 (HCO-60, NIKKOL), which is a surfactant, so that the final concentration was 0.5%, followed by sufficient stirring. Ethanol was then distilled off by a nitrogen gas stream. In order to adjust osmotic pressure, concentrated glycerin was added to this solution so that the final concentration was 1%. Phosphate buffered saline (PBS) was adjusted to pH 8, and added so that the total amount was 100 ml. After the solution was sufficiently stirred, 20 ml was collected and filtered with a 0.22 µmGV filter (Merck Millipore). 5 ml of the first filtrate was discarded, and 15 ml of the subsequent filtrate was collected. 5 ml of this filtrate was placed in each glass test tube for 4°C, 25°C, and 37°C, shaded by a parafilm and an aluminum foil, and preserved under each temperature condition. The stability of this sample was studied by a high performance liquid chromatography (HPLC, Shimadzu Corporation). HPLC conditions were as follows: acetonitrile containing 0.05% trifluoroacetic acid and water were used at the ratio of 60:40 as a mobile phase, the temperature of VR-ODS column (4.6 × 150 mm, Shimadzu Corporation) was set to be 40°C, the flow rate was set to be 1.0 ml/min, the detection wavelength was set to be 254 nm, the injection volume was set to be 10 µl, and the detection time was set to be 10 minutes. In this case, the stability was evaluated over time by assuming the results of HPLC measurement immediately after preparation to be 100% and comparing said results with the results after 2 weeks and 4 weeks. Further, the conditions of pH 7, pH 6.5 and pH 6 were prepared and studied in the same method as described above.

### (Results)

Figure **3** shows the results. Under the condition of 4°C, emricasan which was not degraded was no less than 90% in 4 weeks. However, under the conditions of 25°C and 37°C, degradation of emricasan was observed, which suggested that emricasan would be unstable in a solution at 25°C and 37°C (data not shown). Further, the results show that the higher the pH was, the lower the stability was within the tested range.

### (Example 4: Stability of emricasan using Tween 80)

### (Materials and Methods)

Stability of when Tween 80 was used as a surfactant to dissolve emricasan to a final concentration of 0.5% was tested. 500 mg of emricasan was accurately measured with an electronic balance (AUW220D, SHIMADZU) and transferred to a glass test tube. 2 ml of 99% ethanol was added thereto to dissolve emricasan. The solution thereof was sufficiently stirred with a vortex mixer in order to completely dissolve emricasan. The solution thereof was then added with polysorbate 80 (Tween 80, NIKKOL), which is a surfactant, so that the final concentration was 0.5%, followed by sufficient stirring. Ethanol was then distilled off by a nitrogen gas stream. In order to adjust osmotic pressure, concentrated glycerin was added to this solution so that the final concentration was 1%. Phosphate buffered saline (PBS) was adjusted to pH 8, and added so that the total amount was 100 ml. After the solution was sufficiently stirred, 20 ml was collected and filtered with a 0.22 µmGV filter (Merck Millipore). 5 ml of the first filtrate was discarded, and 15 ml of the subsequent filtrate was collected. 5 ml of this filtrate was placed in each glass test tube for 4°C, 25°C, and 37°C, shaded by a parafilm and an aluminum foil, and preserved under each temperature condition. The stability of this sample was studied by a high performance liquid chromatography (HPLC, Shimadzu Corporation). HPLC conditions were as follows: acetonitrile containing 0.05% trifluoroacetic acid and water were used at the ratio of 60:40 as a mobile phase, the temperature of VR-ODS column (4.6 × 150 mm, Shimadzu Corporation) was set to be 40°C, the flow rate was set to be 1.0 ml/min, the detection wavelength was set to be 254 nm, the injection volume was set to be 10 µl, and the detection time was set to be 10 minutes. In this case, the stability was studied over time by assuming the results of HPLC measurement immediately after preparation to be 100% and comparing said results with the results after 2 weeks and 4 weeks. The conditions of pH 7, pH 6.5 and pH 6 were prepared and studied in the same method as described above.

### (Results)

Figure **4** shows the results. Under the condition of 4°C, emricasan which was not degraded was no less than 90% in 4 weeks. However, under the conditions of 25°C and 37°C, degradation of emricasan was observed, which suggested that emricasan would be unstable in a solution at 25°C and 37°C (data not shown). Further, the results show that the higher the pH was, the lower the stability was. Although these results have the same tendency as the results of using HCO-60 (Example 3), Tween 80 had a tendency to be relatively stable as compared to HCO-60.

### (Example 5: Study of stability in an acidic region)

It is expected that the stability of emricasan, which is an acidic compound, is improved, and emricasan takes a molecular form to improve the intraocular transfer in an acidic region, whereas emricasan takes an ionic form to reduce the intraocular transfer in an alkaline region. Thus, the stability of emricasan in an acidic region was studied.

### (Materials and Methods)

Stability of when Tween 80 was used as a surfactant to dissolve emricasan to a final concentration of 0.1% was tested.

45 mg of emricasan was accurately measured with an electronic balance (AUW220D, SHIMADZU) and transferred to a glass test tube. 0.9 ml of 99% ethanol was added thereto to dissolve emricasan. The solution thereof was sufficiently stirred with a vortex mixer in order to completely dissolve emricasan. The solution thereof was then added with polysorbate 80 (Tween 80, NIKKOL), which is a surfactant, so that the final concentration was 0.5%, followed by sufficient stirring. Ethanol was then distilled off by a nitrogen gas stream. In order to adjust osmotic pressure, concentrated glycerin was added to this solution so that the final concentration was 1%. Phosphate buffered saline (PBS) was adjusted to pH 5.5, and added so that the total amount was 45 ml. After the solution was sufficiently stirred, 20 ml was collected and filtered with a 0.22 µmGV filter (Merck Millipore). 5 ml of the first filtrate was discarded, and 15 ml of the subsequent filtrate was collected. 5 ml of this filtrate was placed in each glass test tube for 4°C, 25°C, and 37°C, shaded by a parafilm and an aluminum foil, and preserved under each temperature condition. The stability of this sample was studied by a high performance liquid chromatography (HPLC, Shimadzu Corporation). HPLC conditions were as follows: acetonitrile containing 0.05% trifluoroacetic acid and water were used at the ratio of 60:40 as a mobile phase, the temperature of VR-ODS column (4.6 × 150 mm, Shimadzu Corporation) was set to be 40°C, the flow rate was set to be 1.0 ml/min, the detection wavelength was set to be 254 nm, the injection volume was set to be 10 µl, and the detection time was set to be 10 minutes. In this case, the stability was studied over time by assuming the results of HPLC measurement immediately after preparation to be 100% and comparing said results with the results after 2 weeks and 4 weeks. Further, the condition of pH 4.5 was prepared and studied in the same method as described above.

### (Results)

Figure **5** shows the results. At the final concentration of 0.1%, emricasan dissolved at both pH 4.5 and pH 5.5. When emricasan was dissolved to the final concentration of 0.1%, emricasan which was not degraded was no less than 90% in 4 weeks at any pH under the condition of 4°C. Emricasan which was not degraded in 2 weeks under the condition of 25°C was less than 90% at pH 5.5, but such emricasan was no less than 50% even after 4 weeks. Meanwhile, emricasan which was not degraded in 2 weeks under the condition of 25°C was no less than 90% at pH 4.5. Further, degradation of emricasan was observed at pH 4.5 and pH 5.5 under the condition of 37°C, which suggested that emricasan would be unstable even under a low pH condition. Thus, the stability was better at pH 4.5 as compared to other pH.

### (Example 6: Lyophilization test of emricasan ophthalmic solution)

It was shown that an emricasan ophthalmic solution would be degraded when preserved at room temperature for a long period of time in the region of pH 6 or greater. In order to prepare emricasan as an eye drop, development of a technique for stabilizing emricasan for a long period of time has an advantage in clinical use. Thus, development of a lyophilized formulation was studied.

### (Materials and Methods)

7 mg of emricasan was accurately measured with an electronic balance (AUW220D, SHIMADZU) and transferred to a glass test tube. 140 µl of 99% ethanol was added thereto to dissolve emricasan. The solution thereof was sufficiently stirred with a vortex mixer in order to completely dissolve emricasan. The solution thereof was then added with polysorbate 80 (Tween 80, NIKKOL), which is a surfactant, so that the final concentration was 0.5%, followed by sufficient stirring. Ethanol was then distilled off by a nitrogen gas stream. D-mannitol (Wako Pure Chemical Industries, Ltd.) was dissolved into phosphate buffered saline (PBS) so that the final concentration was 4.5%, and the solution thereof was adjusted to pH 4.5 and added to the above solution so that the total amount was 7 ml. This solution was sufficiently stirred, and preserved in a -83°C freezer for one day. After freezing, water was completely removed by a lyophilization machine (FDU-1200, TOKYO RIKAKIKAI CO, LTD) for 12 hours.

Water was then added to study the re-solubility. Further, after this sample was filtered with a 0.22 µmGV filter (Merck Millipore), the re-solubility of emricasan was measured by a high performance liquid chromatography (HPLC, Shimadzu Corporation). HPLC conditions were as follows: acetonitrile containing 0.05% trifluoroacetic acid and water were used at the ratio of 60:40 as a mobile phase, the temperature of VR-ODS column (4.6 × 150 mm, Shimadzu Corporation) was set to be 40°C, the flow rate was set to be 1.0 ml/min, the detection wavelength was set to be 254 nm, the injection volume was set to be 10 µl, and the detection time was set to be 10 minutes.

### (Results)

The sample immediately after lyophilization was in a white powder state. After addition of water, the sample was immediately dissolved but was pale white. After filtration with a 0.22 µmGV filter, about 88% of the amount of emricasan before lyophilization was dissolved. Thus, it was found that emricasan can be lyophilized and can withstand re-dissolution with water.

### (Example 7: Stability test of ophthalmic solution prepared from lyophilized emricasan)

### (Materials and Methods)

15 mg of emricasan was accurately measured with an electronic balance (AUW220D, SHIMADZU) and transferred to a glass test tube. 300 µl of 99% ethanol was added thereto to dissolve emricasan. The solution thereof was sufficiently stirred with a vortex mixer in order to completely dissolve emricasan. The solution thereof was then added with polysorbate 80 (Tween 80, NIKKOL), which is a surfactant, so that the final concentration was 0.5%, followed by sufficient stirring. Ethanol was then distilled off by a nitrogen gas stream. D-mannitol (Wako Pure Chemical Industries, Ltd.) was dissolved into phosphate buffered saline (PBS) so that the final concentration was 4.5%, and the solution thereof was adjusted to pH 4.5 and added to the above solution so that the total amount was 15 ml. This solution was sufficiently stirred, and 13 ml was collected and filtered with a 0.22 µmGV filter (Merck Millipore). 4 ml of the first filtrate was discarded, and 8 ml of the subsequent filtrate was collected. 2 ml of this filtrate was placed in each glass test tube for an initial value, 4°C, 25°C, and 37°C, and preserved in a -83°C freezer for one day. After freezing, water was completely removed by a lyophilization machine (FDU-1200, TOKYO RIKAKIKAI CO, LTD) for 12 hours. The lyophilized powder was shaded by a parafilm and an aluminum foil and preserved under each temperature condition. The stability of this sample was studied by a high performance liquid chromatography (HPLC, Shimadzu Corporation). HPLC conditions were as follows: acetonitrile containing 0.05% trifluoroacetic acid and water were used at the ratio of 60:40 as a mobile phase, the temperature of VR-ODS column (4.6 × 150 mm, Shimadzu Corporation) was set to be 40°C, the flow rate was set to be 1.0 ml/min, the detection wavelength was set to be 254 nm, the injection volume was set to be 10 µl, and the detection time was set to be 10 minutes. In this case, the stability was studied over time by assuming the results of HPLC measurement immediately after preparation to be 100% and comparing said results with the results after 2 weeks.

### (Results)

The results measured in this example indicate that the stability is maintained in a prescription in a lyophilized state. Thus, an emricasan ophthalmic solution as a lyophilized formulation can serve as a formulation.

### (Example 8: Examples of preparation of ophthalmic solution)

An ophthalmic solution is provided as lyophilized emricasan and a solvent for reconstitution thereof. Emricasan that is lyophilized immediately before use is mixed with a solvent for reconstitution in an instillation container, thereby preparing an emricasan ophthalmic solution. After preparation of the emricasan ophthalmic solution, it is preserved, for example, at room temperature for 2 weeks to be able to be used. A 0.2% phosphate buffer eye drop base which is adjusted to pH 4.5 is used for the solvent for reconstitution. The solvent for reconstitution may comprise saline, or may further comprise an antiseptic.

### (Example 9: In vivo test)

### (Instillation test on a mouse)

A mouse having a type 8 collagen mutation (col8a2 ^{Q455K/Q433K}) is used as a Fuchs' endothelial corneal dystrophy model mouse. Severity of Fuchs' endothelial corneal dystrophy is graded from a corneal endothelial image of before the instillation test to use Fuchs' endothelial corneal dystrophy model mice that are 20 to 24 weeks old with the same degree of condition. 2 µl of the emricasan ophthalmic solution (0.1% (pH 4.5), 0.5% (pH 7.0)) that is prepared by dissolving lyophilized emricasan with phosphate buffer is instilled into each of the left and right eyes of 45 mice twice a day in the morning and in the evening. Phosphate buffer at pH 4.5 or pH 7.0 is used as a control. The instillation period is 3 months, during which the person in charge of the experimentation carries out the experimentation in a blinded state regarding the emricasan ophthalmic solution and the control ophthalmic solution.

### (Evaluation of the effectiveness of the ophthalmic solution)

Before starting the instillation test, corneal endothelial images are observed with a contact corneal endothelial specular microscope for grading. After starting the instillation test, the corneal endothelial images of the mice are observed once every 4 weeks with the contact corneal endothelial specular microscope to evaluate the effectiveness of the emricasan ophthalmic solution.

### (Expected results)

It is expected that the decrease in corneal endothelial cell density, observed using the contact-type corneal endothelial specular microscope, in Fuchs' endothelial corneal dystrophy model mice is suppressed in the individuals to which the emricasan ophthalmic solution was administered, compared to the control. Furthermore, it is expected that the percentage of the area of guttae is decreased in the individuals administered with the emricasan ophthalmic solution compared to the control.

As disclosed above, the present disclosure is exemplified by the use of its preferred embodiments of the present disclosure. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2018-125234, which was filed on June 29, 2018. The entire content thereof is incorporated herein by reference.

### [Industrial Applicability]

A formulation wherein emricasan is solubilized/stabilized is provided. Emricasan is expected to have a therapeutic effect on corneal endothelial disorders. In particular, techniques that are available in the technical field relating to ophthalmic formulations are provided.

## Claims

1. A formulation comprising:
(i) emricasan or a derivative thereof, or a pharmaceutically acceptable salt thereof, or a solvate thereof;
(ii) a nonionic surfactant comprising a polyoxyethylene group; and
(iii) a buffering agent that maintains pH at about 3.0 to about 8.5.

2. The formulation of claim 1, wherein the buffering agent maintains pH at about 3.5 to about 8.0.

3. The formulation of claim 2, wherein the buffering agent maintains pH at about 4.0 to about 7.0.

4. The formulation of claim 2, wherein the buffering agent maintains pH at about 4.5 to about 6.5.

5. The formulation of any one of claims 1 to 4, wherein the surfactant is present in the formulation at about 0.05% by weight to about 5% by weight.

6. The formulation of any one of claims 1 to 4, wherein the surfactant is present in the formulation at about 0.1% by weight to about 4% by weight.

7. The formulation of any one of claims 1 to 6, wherein the nonionic surfactant comprising a polyoxyethylene group is selected from the group consisting of polyoxyethylene hydrogenated castor oil, polyoxyethylene castor oil, polyethylene glycol monostearate, polyoxyethylene sorbitan fatty acid ester, and polyoxyethylene-polyoxypropylene copolymer.

8. The formulation of any one of claims 1 to 7, wherein the nonionic surfactant comprising a polyoxyethylene group is selected from the group consisting of HCO-20, HCO-40, HCO-60, HCO-80, HCO-100, CO-35, MYS-40, Tween 80, and poloxamer 407.

9. The formulation of any one of claims 1 to 8, wherein the emricasan or a derivative thereof, or a pharmaceutically acceptable salt thereof, or a solvate thereof is present in the formulation at about 0.05% by weight to about 5% by weight.

10. The formulation of any one of claims 1 to 8, wherein the emricasan or a derivative thereof, or a pharmaceutically acceptable salt thereof, or a solvate thereof is present in the formulation at about 0.1% by weight to about 1% by weight.

11. A formulation comprising emricasan, wherein the formulation comprises:
(i) emricasan that is present in the formulation at about 0.1% by weight to about 1% by weight;
(ii) a surfactant selected from the group consisting of HCO-20, HCO-40, HCO-60, HCO-80, HCO-100, CO-35, MYS-40, Tween 80, and poloxamer 407, the surfactant being present in the formulation at about 0.1% by weight to about 4% by weight; and
(iii) a buffering agent that maintains pH at about 3.0 to about 8.0.

12. A formulation comprising emricasan, wherein the formulation comprises:
(i) emricasan that is present in the formulation at about 0.1% by weight to about 1% by weight;
(ii) HCO-60 that is present in the formulation at about 0.1% by weight to about 4% by weight; and
(iii) a buffering agent that maintains pH at about 4.0 to about 7.0.

13. A formulation comprising emricasan, wherein the formulation comprises:
(i) emricasan that is present in the formulation at about 0.1% by weight to about 1% by weight;
(ii) Tween 80 that is present in the formulation at about 0.1% by weight to about 4% by weight; and
(iii) a buffering agent that maintains pH at about 4.0 to about 7.0.

14. The formulation of claims 1 to 13, wherein the formulation optionally further comprises a saccharide.

15. The formulation of claim 14, wherein the saccharide is selected from the group consisting of mannitol, sucrose, and trehalose.

16. The formulation of any one of claims 1 to 15, wherein the formulation is an ophthalmic formulation.

17. The formulation of claim 16, wherein the formulation is a formulation for treating or preventing a corneal endothelial condition, disorder, or disease.

18. The formulation of any one of claims 1 to 17, wherein the formulation is a formulation for instillation or a formulation for injection into an anterior chamber.

19. The formulation of any one of claims 1 to 18, wherein the formulation is an aqueous solution.

20. A composition for preparing the formulation of claim 19 at the time of use, comprising emricasan or a derivative thereof, or a pharmaceutically acceptable salt thereof, or a solvate thereof, wherein the composition is **characterized by** being in a powder form and being reconstituted with a medium for reconstitution.

21. The composition of claim 20, wherein the composition comprises:
(i) emricasan or a derivative thereof, or a pharmaceutically acceptable salt thereof, or a solvate thereof;
(ii) a nonionic surfactant comprising a polyoxyethylene group; and
(iii) a buffering agent that maintains pH at about 3.0 to about 8.5.

22. The composition of claim 20 or 21, wherein the medium for reconstitution comprises purified water, saline, buffer, or any combination thereof.

23. The composition of any one of claims 20 to 22, wherein the solvent for reconstitution further comprises at least one selected from the group consisting of an antiseptic, a stabilizer, and an isotonicifier.

24. The composition of any one of claims 20 to 23, wherein the powder form is a lyophilized powder form.

25. The composition of any one of claims 20 to 24, wherein the medium for reconstitution comprises a nonionic surfactant comprising a polyoxyethylene group.

26. The composition of any one of claims 20 to 25, wherein the medium for reconstitution comprises a buffering agent that maintains pH at about 3.0 to about 8.5.

27. The composition of claims 20 to 26, wherein the medium for reconstitution optionally further comprises a saccharide.

28. The composition of claim 27, wherein the saccharide is selected from the group consisting of mannitol, sucrose, and trehalose.

29. A kit for preparing the formulation of claim 19 at the time of use, wherein the kit comprises:
(i) powder which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5; and
(ii) a solvent for reconstitution.

30. The kit of claim 29, wherein the powder is powder obtained by lyophilizing a solution which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5.

31. The kit of claim 29 or 30, wherein the medium for reconstitution comprises purified water, saline, buffer, or any combination thereof.

32. The kit of any one of claims 29 to 31, wherein the medium for reconstitution further comprises at least one selected from the group consisting of an antiseptic, a stabilizer, and an isotonicifier.

33. A kit for preparing the formulation of claim 19 at the time of use, wherein the kit comprises:
(i) powder of emricasan or a derivative thereof, or a pharmaceutically acceptable salt thereof, or a solvate thereof;
(ii) optionally a nonionic surfactant comprising a polyoxyethylene group; and
(iii) optionally a buffering agent that maintains pH at about 3.0 to about 8.5.

34. A kit for preparing the formulation of claim 17 at the time of use, wherein the kit comprises:
(i) powder of emricasan or a derivative thereof, or a pharmaceutically acceptable salt thereof, or a solvate thereof;
(ii) a medium for reconstitution which comprises a nonionic surfactant comprising a polyoxyethylene group and a buffering agent that maintains pH at about 3.0 to about 8.5.

35. The kit of claim 33 or 34, wherein the powder is lyophilized powder.

36. The kit of claims 29 to 35, wherein the medium for reconstitution optionally further comprises a saccharide.

37. The kit of claim 36, wherein the saccharide is selected from the group consisting of mannitol, sucrose, and trehalose.

38. A method for preparing the formulation of claim 19 at the time of use, comprising:
(i) providing powder which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5; and
(ii) reconstituting the powder with a medium for reconstitution.

39. The method of claim 38, wherein the powder is powder obtained by lyophilizing a solution which comprises emricasan or a derivative thereof, or a pharmaceutically acceptable salt thereof, or a solvate thereof, a nonionic surfactant comprising a polyoxyethylene group, and a buffering agent that maintains pH at about 3.0 to about 8.5.

40. The method of claim 38 or 39, wherein the medium for reconstitution comprises purified water, saline, buffer, or any combination thereof.

41. The method of any one of claims 38 to 40, wherein the medium for reconstitution further comprises at least one selected from the group consisting of an antiseptic, a stabilizer, and an isotonicifier.

42. A method for preparing the formulation of claim 19 at the time of use, comprising:
(i) providing powder of emricasan or a derivative thereof, or a pharmaceutically acceptable salt thereof, or a solvate thereof; and
(ii) reconstituting the powder with a medium for reconstitution which comprises a nonionic surfactant comprising a polyoxyethylene group and a buffering agent that maintains pH at about 6.0 to about 8.5.

43. The method of claim 42, wherein the powder is lyophilized powder.

44. The method of claim 42 or 43, wherein the solvent for reconstitution optionally further comprises a saccharide.

45. The method of claim 44, wherein the saccharide is selected from the group consisting of mannitol, sucrose, and trehalose.

46. A medium or solution for reconstituting lyophilized powder of emricasan or a derivative thereof, or a pharmaceutically acceptable salt thereof, or a solvate thereof, or for dissolving a crystal or powder thereof of emricasan or a derivative thereof, or a pharmaceutically acceptable salt thereof, or a solvate thereof, wherein the medium or solution comprises:
(i) a solvent;
(ii) a nonionic surfactant comprising a polyoxyethylene group; and
(iii) a buffering agent that maintains pH at about 3.0 to about 8.5.

47. The medium or solution of claim 46, wherein the medium or solution optionally further comprises a saccharide.

48. The medium or solution of claim 47, wherein the saccharide is selected from the group consisting of mannitol, sucrose, and trehalose.
